# EUROPEAN PATENT APPLICATION

(11) **EP 4 316 425 A1**
(43) Date of publication of application: **07.02.2024**
(21) Application number: 21932779.8
(22) Date of filing: 24.12.2021
(51) Int. Cl.: A61F 2/24

(54) **ARTIFICIAL HEART VALVE**

(30) Priority: 26.03.2021 CN 202110326678
(71) Applicant: Shanghai Shenqi Medical Technology Co., Ltd., Shanghai 201203 (CN)
(72) Inventor: WANG, Sen, Shanghai 201203 (CN); ZHOU, Guolei, Shanghai 201203 (CN); JIANG, Jianjian, Shanghai 201203 (CN); DAI, Zhihao, Shanghai 201203 (CN)
(74) Representative: Cabinet Laurent & Charras
(86) International application number: PCT/CN2021/141262
(87) International publication number: WO 2022/199172

(57) **Abstract**

An artificial heart valve, comprising a grid tube body (1), a first conical net rack (21) and a second conical net rack (22) sequentially sleeved on a same end of the grid tube body (1) in the axial direction of the grid tube body (1), and covering films respectively provided on the grid tube body (1), the first conical net rack (21) and the second conical net rack (22). The conical surface of the first conical net rack (21) and the conical surface of the second conical net rack (22) are arranged opposite to each other to clamp the inner wall of the heart located between the first conical net rack (21) and the second conical net rack (22), and the covering films located on the first conical net rack (21) and the second conical net rack (22) respectively form a first sealing surface and a second sealing surface together with the inner wall of the heart.

## Description

### CROSS-REFERENCE TO RELATED APPLICATIONS

The present application claims priority to Chinese patent application No. 2021103266789, filed on March 26, 2021, the disclosure of which is hereby incorporated by reference in its entirety.

### TECHNICAL FIELD

The present application relates to a technical field of medical devices, in particular, to an artificial heart valve.

### BACKGROUND

The prevalence of valvular heart disease increases significantly with the aging of the population. According to survey data, moderate or severe valvular diseases were found in 615 of 11,911 randomly selected adults, and the prevalence increased with age, from 0.7% in the 18-44 age group to 13.2% in the 75 and older age group.

Currently, the main clinical treatments for valve-related diseases are repair (including valve ring repair, tendon repair, valve leaflet repair, etc.) and replacement. Valve replacement is categorized into surgical replacement and minimally invasive transcatheter replacement according to the type of clinical procedure.

A conventional surgical replacement requires a surgery to open the patient's heart through. On the one hand, this surgical method will cause great pain to the patient, and even if the surgery goes well, it will inevitably leave postoperative sequelae due to the open chest treatment; on the other hand, due to the fact that most of the valve-related diseases are found in middle-aged and elderly patients, and most middle-aged and elderly patients are physically limited, the conventional surgical treatment is not allowed.

Minimally invasive transcatheter replacement of artificial heart valve is a new approach of valve replacement treatment in recent years. Compared with the conventional surgical replacement, this method does not require open chest treatment, which greatly reduces the patient's pain and the occurrence of complications and sequelae, and at the same time, which also brings a new hope for the treatment to critically ill patients who cannot undergo conventional surgical treatment.

However, due to the complexity of the structure of the mitral valve in the human body, the artificial heart valve implanted through a catheter does not fit the heart satisfactorily when it is fixed to the corresponding position in the heart, which can cause blood in the left ventricle to flow back to the left atrium through the position where the artificial heart valve is fixed to the heart. This may cause arrhythmia and heart failure, and in severe cases, may even lead to sudden death.

There is therefore an urgent need to address the above issues.

### SUMMARY

According to various embodiments of the present application, an artificial heart valve is provided.

In an aspect of the application, an artificial heart valve is provided. The artificial heart valve includes: a mesh tube body; a first conical mesh frame and a second conical mesh frame sequentially sleeved on a same end of the mesh tube body in an axial direction of the mesh tube body; and cover films respectively disposed on the mesh tube body, the first conical mesh frame, and the second conical mesh frame. A conical surface of the first conical mesh frame and a conical surface of the second conical mesh frame are disposed opposite to each other, so as to clamp an inner wall of a heart located between the first conical mesh frame and the second conical mesh frame, and enable the cover films disposed on the first conical mesh frame and the second conical mesh frame to respectively form a first sealing surface and a second sealing surface with the inner wall of the heart.

In an embodiment, an angle between the conical surface of the first conical mesh frame and a horizontal plane is in a range from 0 to 80 degrees, and an angle between the conical surface of the second conical mesh frame and the horizontal plane is in a range from 0 to 80 degrees.

In an embodiment, the second conical mesh frame includes a first mesh frame body and a second mesh frame body. The first mesh frame body has a first taper, and the second mesh frame body has a second taper. An angle between a conical surface of the first mesh frame body and the horizontal plane is in a range from 0 to 80 degrees. An angle between a conical surface of the second mesh frame body and the horizontal plane is approximately 90 degrees. A center angle α of the second mesh frame body is in a range from 50 to 180 degrees.

In an embodiment, the first conical mesh frame and the second conical mesh frame are integrally formed.

In an embodiment, the second conical mesh frame is provided with barbs. An acute angle is formed between the barbs and the conical surface of the second conical mesh frame.

In an embodiment, a grid of the mesh tube body has a rhombic shape, and a vertex angle of the rhombic shape is in range from 37 to 100 degrees.

In an embodiment, the artificial heart valve has a total length of 10 to 35 mm.

In an embodiment, the second conical mesh frame has a D-shaped cross-section.

In an embodiment, another end of the mesh tube body is provided with discrete connecting portions in a circumferential array.

In an embodiment, 2 to 12 connecting portions are provided.

In an embodiment, a stress relief hole is defined on a connecting body between two adjacent grids on the mesh tube body.

By disposing the conical surface of the first conical mesh frame and the conical surface of the second conical mesh frame to be opposite to each other, to clamp the inner wall of the heart located between the first conical mesh frame and the second conical mesh frame, and making the cover films disposed on the first conical mesh frame and the second conical mesh frame respectively form the first sealing surface and the second sealing surface with the inner wall of the heart, problems such as arrhythmia as well as heart failure due to the blood in the left ventricle flowing back into the left atrium through the position where the artificial heart valve is fixed to the heart can be prevented, thereby improving the success rate of the surgery.

### BRIEF DESCRIPTION OF THE DRAWINGS

In order to more clearly illustrate the technical solutions in the embodiments of the present application or the prior art, a brief description is given below for the drawings referred in the description of the embodiments or the prior art. Obviously, the drawings in the following description are merely some embodiments of the application. For those of ordinary skill in the art, other drawings can also be obtained based on these drawings without involving any inventive effort.
FIG. 1 is a schematic diagram of a structure of an artificial heart valve according to an embodiment of the present application.
FIG. 2 is a schematic diagram of a structure of a transplanted artificial heart valve according to an embodiment of the present application.
FIG. 3 is a front view of FIG. 1.
FIG. 4 is a cross-sectional schematic diagram taken along a line A-A in FIG. 3.
FIG. 5 is a schematic diagram of a structure of a first conical mesh frame and a second conical mesh frame shown in FIG. 1.
FIG. 6 is a front view of FIG. 5;
FIG. 7 is a top view of the structure shown in FIG. 5.
FIG. 8 is a front view of a mesh tube body shown in FIG. 1.
FIG. 9 is a partially enlarged view of a portion R in FIG. 8.

References Numerals:
1, mesh tube body; 11, connecting portion; 12, stress relief hole;
21, first conical mesh frame; 22, second conical mesh frame; 221, first mesh frame body; 222, second mesh frame body; 223, inverted thorn;
31, first cover film; 32, second cover film; 33, third cover film;
4, valve leaflet;
51, left atrium; 52, left ventricle; 53, aorta; 541, first native valve leaflet; and 542, second native valve leaflet.

### DETAILED DESCRIPTION

The following will provide a further detailed explanation of the present application in conjunction with the drawings and embodiments. It should be understood that specific embodiments described herein are only intended to explain the application but not intended to limit the application. It should be noted that, for the convenience of description, only parts, and not all, of the structure related to the present application are shown in the drawings.

In the description of the present application, unless otherwise expressly specified and defined, the terms "connected", "coupled" and "fixed" should be understood in a broad sense, for example, may be fixedly connected or detachably connected, or integrated; or mechanically connected or electrically connected; or directly connected or indirectly connected through an intermediate medium, or in an interior communication or mutual interaction relationship between two elements. For those of ordinary skill in the art, the specific meanings of the above-described terms in the present application may be understood according to specific circumstances.

In the present application, unless otherwise expressly specified and defined, the first feature, when being referred to as being "on" or "under" the second feature, may be in direct contact with the second feature, or may be in indirect contact with the second feature through other features therebetween. Moreover, the first feature, when being referred to as being disposed "on", "above" and "over" the second feature, may be disposed right above or obliquely above the second feature, or simply disposed at a level higher than the second feature. The first feature, when being referred to as being disposed "under", "below" and "beneath" the second feature, may be disposed directly below or obliquely below the second feature, or simply disposed at a level lower than the second feature.

In the description of the embodiments, it should be understood that the terms "up", "down", "right", etc., indicate the orientations or position relationships based on the orientations or position relationships shown in the drawings, and are used only for the convenience of describing and simplifying operations, rather than indicating or implying that a device or an element referred to must have a specific orientation, be constructed and operated in a specific orientation, therefore they should not be construed as limitation on the present application. In addition, the terms "first" and "second" are only used to distinguish descriptors and have no special meaning.

Artificial heart valves can be implanted in a heart to replace diseased heart valves. A mitral valve (i.e., left atrioventricular valve) is attached to a fibrous ring around a periphery of a left atrioventricular orifice and has two valvulae. During a contraction of a left ventricle, the mitral valve closes the atrioventricular orifice to prevent backflow of blood from the left ventricle into a left atrium.

Minimally invasive transcatheter replacement of artificial heart valve is a new approach of valve replacement in recent years. Instead of opening the chest, the artificial heart valve is compressed into a delivery sheath, passed through a blood vessel to arrive at a corresponding position in the heart, and then the compressed artificial heart valve is released from the delivery sheath.

Existing artificial heart valves are provided with barbs and thus secured to the fibrous ring around the periphery of the left atrioventricular orifice. However, the artificial heart valves secured by the barbs does not fit the heart satisfactorily, which can cause blood in the left ventricle to flow back to the left atrium through a gap formed between the heart and the artificial heart valve fixed to the heart. This may cause arrhythmia and heart failure, and in severe cases, may even lead to sudden death.

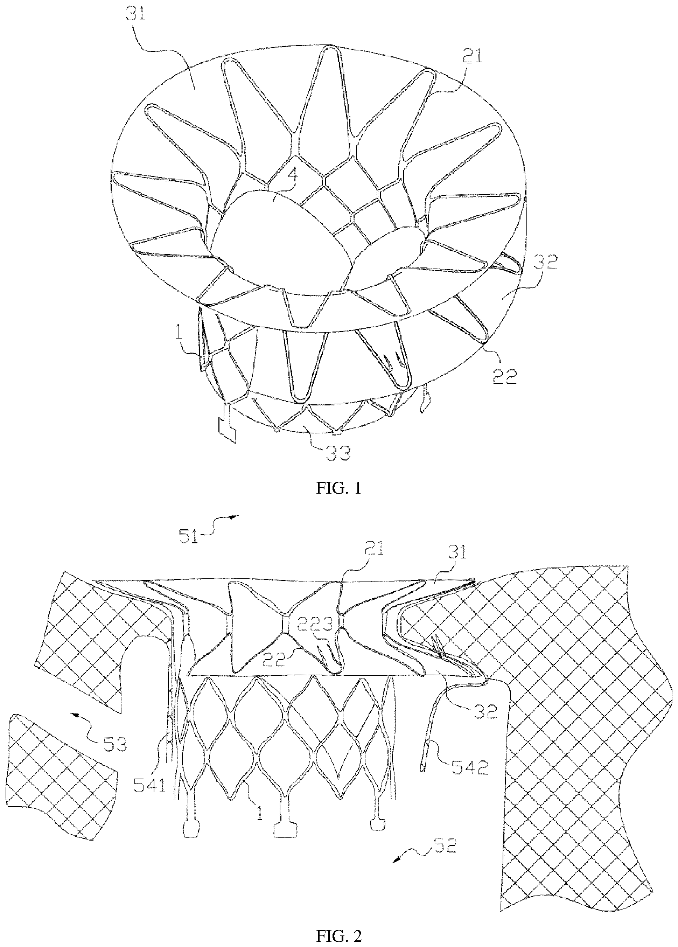

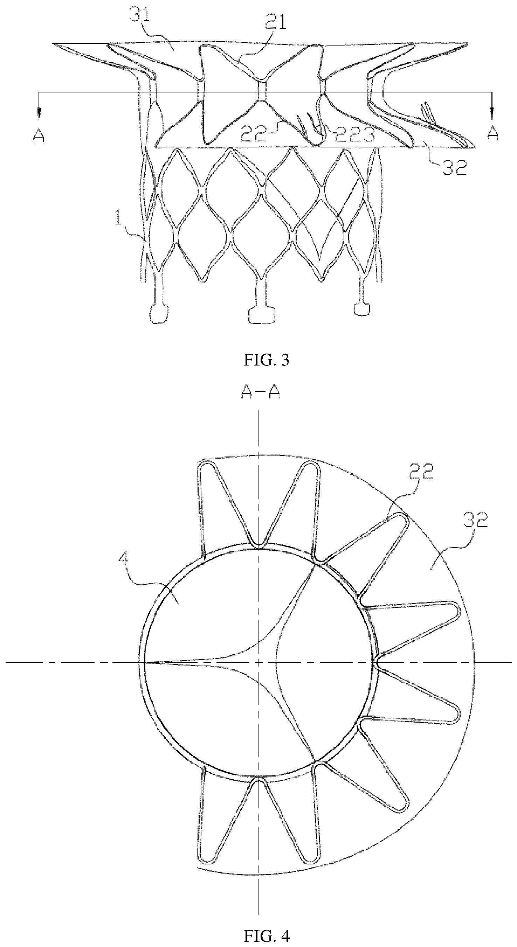

In order to solve the above problem, the present application provides an artificial heart valve, as shown in FIG. 1 and FIG. 3, which includes a mesh tube body 1, a first conical mesh frame 21, a second conical mesh frame 22, a cover film, and a valve leaflet 4. The first conical mesh frame 21 and the second conical mesh frame 22 are sequentially sleeved on a same end of the mesh tube body 1 in an axial direction of the mesh tube body 1, in order to fix the artificial heart valve to the fibrous ring around the periphery of the left atrioventricular orifice.

The cover film includes a first cover film 31 disposed on the first conical mesh frame 21, a second cover film 32 disposed on the second conical mesh frame 22, and a third cover film 33 disposed on the mesh tube body 1, so as to enable the artificial heart valve to form a channel from one end of the mesh tube body 1 to another end of the mesh tube body 1.

The valve leaflets 4 are disposed in the channel. When the left ventricle 52 contracts, the heart compresses the blood in the left ventricle 52, and the blood impact the valve leaflets 4, causing the valve leaflets 4 to close the channel so that the blood does not flow into the left atrium 51.

The artificial heart valve, after being compressed, is delivered to the heart via a delivery sheath, and the released artificial heart valve undergoes an elastic deformation in order to return its initial state. By disposing a conical surface of the first conical mesh frame 21 and a conical surface of the second conical mesh frame 22 to be opposite to each other, the first conical mesh frame 21 and the second conical mesh frame 22 clamp the fibrous ring around the periphery of the left atrioventricular orifice. The cover film disposed on the first conical mesh frame 21 and the cover film disposed on the second conical mesh frame 22 respectively form a first sealing surface and a second sealing surface with the fibrous ring around the periphery of the left atrioventricular orifice, In this way, the blood in the left ventricle 52 can be prevented from flowing back to the left atrium 51 through between the artificial heart valve and the fibrous ring around the periphery of the left atrioventricular orifice when the left ventricle 52 contracts, so as to prevent problems such as arrhythmia as well as heart failure due to the blood in the left ventricle 52 flowing back into the left atrium 51 through the position where the artificial heart valve is fixed to the heart, thereby improving the success rate of the surgery.

Furthermore, in order to enable the first conical mesh frame 21 and the second conical mesh frame 22 to stably and securely clamp and thus be disposed on the fibrous ring around the periphery of the left atrioventricular orifice, an angle between the conical surface of the first conical mesh frame 21 and a horizontal plane is in a range from 0 to 80 degrees, and an angle between the conical surface of the second conical mesh frame 22 and the horizontal plane is in a range from 0 to 80 degrees.

As the first conical mesh frame 21 and the second conical mesh frame 22 clamp the fibrous ring around the periphery of the left atrioventricular orifice, the second conical mesh frame 22 brings a first native valve leaflet 541 and a second native valve leaflet 542 of the heart close to a peripheral wall of the left ventricle 52, which may result in the first native valve leaflet 541 blocking a channel from the left ventricle 52 to an aorta 53, and thus may block the flow of blood from the left ventricle 52 into the aorta 53, thereby resulting in insufficient blood supply throughout the body.

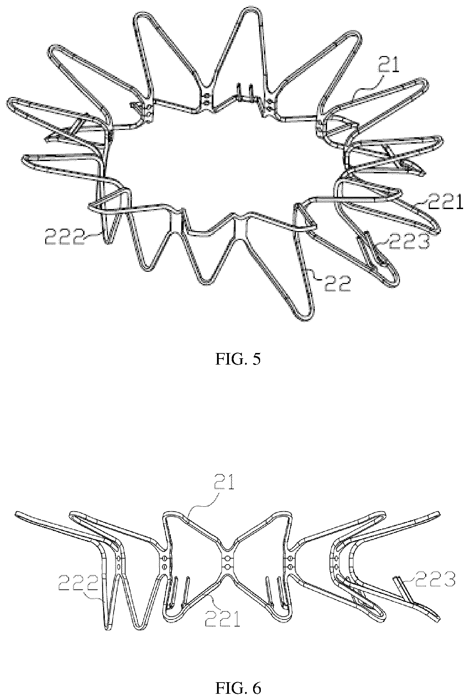

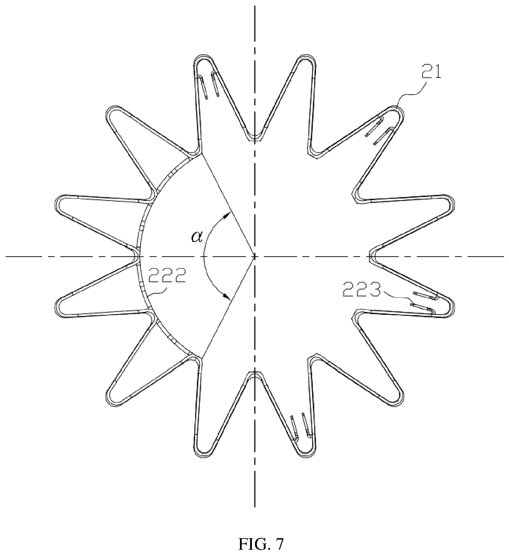

In order to solve the above problem, further, as shown in FIG. 2 to FIG. 6, in the present embodiment, the second conical mesh frame 22 includes a first mesh frame body 221 and a second mesh frame body 222. The first mesh frame body 221 has a first taper, and the second mesh frame body 222 has a second taper. An angle between the conical surface of the second conical mesh frame 22 having the first taper, i.e., the conical surface of the first mesh frame body 221, and the horizontal plane is in a range from 0 to 80 degrees. An angle between the conical surface of the second conical mesh frame 22 having the second taper, i.e., the conical surface of the second mesh frame body 222, and the horizontal plane is approximately 90 degrees. As shown in FIG. 7, a center angle α of the second conical mesh frame 22 having the second taper, i.e., the center angle α of the second mesh frame body 222 is in a range from 50 to 180 degrees. With the above configuration, the first native valve leaflet 541 is away from the channel from the left ventricle 52 to the aorta 53, thereby preventing the first native valve leaflet 541 from blocking the flow of blood into the aorta 53.

Further, in order to improve the strength of the artificial heart valve and the ability of the artificial heart valve to stably and securely clamp and thus disposed on the fibrous ring around the periphery of the left atrioventricular orifice, the first conical mesh frame 21 and the second conical mesh frame 22 are integrally formed, which not only can improve the strength of the artificial heart valve, but also can stably and securely clamp and thus disposed on the fibrous ring around the periphery of the left atrioventricular orifice, so as to improve the sealing property of the artificial heart valve.

Further, in order to enable the artificial heart valve to be more stably and securely fixed to the fibrous ring around the periphery of the left atrioventricular orifice, the second conical mesh frame 22 is provided with barbs 223. An acute angle is formed between the barbs 223 and the conical surface of the second conical mesh frame 22, so that the barbs 223 can hook the fibrous ring around the periphery of the left atrioventricular orifice clamped by the first conical mesh frame 21 and the second conical mesh frame 22, thereby more stably and securely fixing the artificial heart valve.

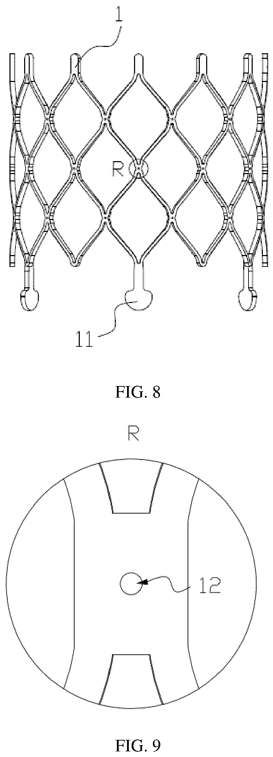

Further, in order to enable the artificial heart valve to be compressed into the delivery sheath and to provide sufficient support force after being released, as shown in FIGS. 3, 8, and 9, the grid of the mesh tube body 1 has a rhombic shape, and the vertex angle of the rhombic shape is in a range from 37 to 100 degrees, thereby enabling the artificial heart valve to be compressed into the delivery sheath and to provide sufficient support force after being released.

Further, in order to prevent the channel from the left ventricle 52 to the aorta 53 from being blocked or obstructed due to the length of the artificial heart valve being too long, the artificial heart valve in this embodiment has a total length of 10 to 35 mm.

Further, since the cross-section of the fibrous ring around the periphery of the left atrioventricular orifice is D-shaped, in order to prevent that the artificial heart valve, after being implanted into the native mitral valve of the heart, may cause compression on a flat sidewall of an accommodation space, which results in the narrowing of an outflow tract of the heart, in this embodiment, the second conical mesh frame 22 has a D-shaped cross-section.

Further, during delivery of the artificial heart valve from the delivery sheath to the heart, in order to facilitate entry or exit of the artificial heart valve into or out of the delivery sheath, the other end of the mesh tube body 1 is provided with discrete connecting portions 11 in a circumferential array. 2 to 12 connecting portions 11 are provided. In this way, the force on the artificial heart valve is uniform, and it can facilitate entry or exit of the compressed artificial heart valve into or out of the delivery sheath.

Further, since the blood flowing from the left ventricle 52 into the aorta 53 is oxygen-rich and high-pressure blood, a certain impact force on the mesh tube body 1 can be caused. In order to reduce the impact on the mesh tube body 1, the connecting portions 11 are retractable in a radial direction of the mesh tube body 1, so as to reduce the impact of the impact force on the mesh tube body 1.

Further, in order to enhance the strength of the mesh tube body 1, stress relief holes 12 are defined on a connecting body between two adjacent grids on the mesh pipe body 1. The stress relief holes 12 can disperse the stress on a center portion of the connecting body between the two adjacent grids, thereby avoiding the phenomenon of stress concentration on the center portion of the connecting body, and enhancing the strength of the mesh tube body 1.

Further, in this embodiment, the mesh tube body 1, the first conical mesh frame 21, and the second conical mesh frame 22 can be integrally formed from nickel titanium alloy with super elasticity and memory properties, or separately formed and then welded, riveted, or bonded.

The cover film may be made of a polymer material such as polytetrafluoroethylene. The first cover film 31 may be secured on the first conical mesh frame 21, the second cover film 32 may be secured on the second conical mesh frame 22, and the third cover film 33 may be secured on the mesh tube body 1, by sewing, bonding, or ultrasonic welding, or the like.

The valve leaflet 4 may be made of bovine pericardium, porcine pericardium, or other polymer films, and may be sewn into the channel of the mesh tube body 1 by sutures.

Obviously, the foregoing embodiments of the present application are merely examples for the purpose of clearly illustrating the present application, and are not intended to limit the embodiments of the present application. Various obvious changes, readjustments, and replacements can be made by those skilled in the art without departing from the scope of protection of the present application. It is neither necessary nor possible to exhaust all the embodiments herein. Any modifications, equivalent replacements, improvements made within the spirit and principles of the present application shall be subject to the protection scope of the claims in this application.

## Claims

1. An artificial heart valve, **characterized by**, comprising:
a mesh tube body (1);
a first conical mesh frame (21) and a second conical mesh frame (22) sequentially sleeved on a same end of the mesh tube body (1) in an axial direction of the mesh tube body (1); and
cover films respectively disposed on the mesh tube body (1), the first conical mesh frame (21) and the second conical mesh frame (22),
wherein a conical surface of the first conical mesh frame (21) and a conical surface of the second conical mesh frame (22) are disposed opposite to each other, so as to clamp an inner wall of a heart located between the first conical mesh frame (21) and the second conical mesh frame (22), and enable the cover films disposed on the first conical mesh frame (21) and the second conical mesh frame (22) to respectively form a first sealing surface and a second sealing surface with the inner wall of the heart.

2. The artificial heart valve according to claim 1, wherein an angle between the conical surface of the first conical mesh frame (21) and a horizontal plane is in a range from 0 to 80 degrees, and an angle between the conical surface of the second conical mesh frame (22) and the horizontal plane is in a range from 0 to 80 degrees.

3. The artificial heart valve according to claim 2, wherein the second conical mesh frame (22) comprises a first mesh frame body (221) and a second mesh frame body (222), and wherein the first mesh frame body (221) has a first taper, the second mesh frame body (222) has a second taper; an angle between a conical surface of the first mesh frame body (221) and the horizontal plane is in a range from 0 to 80 degrees, an angle between a conical surface of the second mesh frame body (222) and the horizontal plane is approximately 90 degrees; and a center angle α of the second mesh frame body (222) is in a range from 50 to 180 degrees.

4. The artificial heart valve according to claim 3, wherein the first conical mesh frame (21) and the second conical mesh frame (22) are integrally formed.

5. The artificial heart valve according to claim 1, wherein the second conical mesh frame (22) is provided with barbs (223), an acute angle is formed between the barbs (223) and the conical surface of the second conical mesh frame (22).

6. The artificial heart valve according to claim 1, wherein a grid of the mesh tube body (1) has a rhombic shape, and a vertex angle of the rhombic shape is in range from 37 to 100 degrees.

7. The artificial heart valve according to claim 1, wherein the artificial heart valve has a total length of 10 to 35 mm.

8. The artificial heart valve according to claim 1, wherein the second conical mesh frame (22) has a D-shaped cross-section.

9. The artificial heart valve according to claim 1, wherein another end of the mesh tube body (1) is provided with discrete connecting portions (11) in a circumferential array.

10. The artificial heart valve according to claim 9, wherein 2 to 12 connecting portions (11) are provided.

11. The artificial heart valve according to claim 1, wherein a stress relief hole (12) is defined on a connecting body between two adjacent grids on the mesh tube body (1).
